# EUROPEAN PATENT APPLICATION

(11) **EP 4 342 510 A1**
(43) Date of publication of application: **27.03.2024**
(21) Application number: 22833017.1
(22) Date of filing: 24.06.2022
(51) Int. Cl.: A61M 11/00, A61M 5/315

(54) **SYRINGE**

(30) Priority: 29.06.2021 JP 2021107729
(71) Applicant: Taisei Kako Co., Ltd., Kita-ku Osaka-shi Osaka 531-0072 (JP)
(72) Inventor: OGAWA, Yukihiro, Ibaraki-shi, Osaka 567-0054 (JP); NISHIKURA, Hidenari, Ibaraki-shi, Osaka 567-0054 (JP); TANIGUCHI, Kensuke, Ibaraki-shi, Osaka 567-0054 (JP); KATAGIRI, Yuki, Ibaraki-shi, Osaka 567-0054 (JP); SASAKI, Ayako, Ibaraki-shi, Osaka 567-0054 (JP); OGAMI, Takashi, Ibaraki-shi, Osaka 567-0054 (JP); NAKAKUBO, Yutaro, Ibaraki-shi, Osaka 567-0054 (JP)
(74) Representative: DTS Patent- und Rechtsanwälte Schnekenbühl und Partner mbB
(86) International application number: PCT/JP2022/025260
(87) International publication number: WO 2023/276870

(57) **Abstract**

The present invention includes: a barrel; a rod; a barrel side member; a rod side member attached to the rod and configured to be shifted between a first state and a second state of being located on a proximal side relative to the first state; and a biasing member configured to bias the rod side member to move it from the first state toward the second state, the rod side member including: a lock part configured to be locked to the rod in the first state; a first pushing part configured to be pushed against the rod toward the distal side in the first state, and be a part separate from the lock part; and a second pushing part configured to be pushed against the rod toward the distal side in the second state, and be a part separate from the lock part; the rod including a first lock part; a first pushed part; and a second pushed part, and the barrel side member including: a lock-release part configured to release lock between the lock part and the first lock part with the rod pushed to the predetermined position in the barrel in the first state.

## Description

### CROSS-REFERENCE TO RELATED APPLICATION

This application claims priority to Japanese Patent Application No. 2021-107729, the disclosure of which is incorporated herein by reference in its entirety.

### FIELD

The present invention relates to a syringe enabling the administration of a medical liquid filled in the syringe into a nasal cavity or an oral cavity.

### BACKGROUND

Conventionally known as a container for dividing a medical liquid for administration into the nasal cavity or the oral cavity of a patient is a nasal cavity administration container capable of dividing a medical liquid to inject the same into both nostrils has been known (Patent Literature 1). As shown in Fig. 9, the nasal cavity administration container 100 includes a barrel 101 to be filled with a medical liquid, and a plunger 102 insertable into the barrel 101. The nasal cavity administration container 100 further includes a plunger rod 103 insertable into the plunger 102, and an elastic member 105 disposed at a front end of the plunger rod 103 in the plunger 102. A projecting part 1016 projects from an inner circumferential surface of the barrel 101. The plunger 102 has a first elongated hole 1026 and a second elongated hole 1028. The plunger rod 103 includes a leaf spring 1030L and a pushing part 1034 located at a rear end of the plunger rod 103. The plunger rod 103 further includes a first lock part 1036 and a second lock part 1038 each disposed in the leaf spring 1030L.

When the medical liquid is administered using the nasal cavity administration container 100, the nasal cavity administration container 100 is shifted from a first administration start state (Fig. 10) to a first administration completion state (Fig. 11), a pressure release state (Fig. 12), a second administration start state (Fig. 13), and a second administration completion state (Fig. 14) in this order.

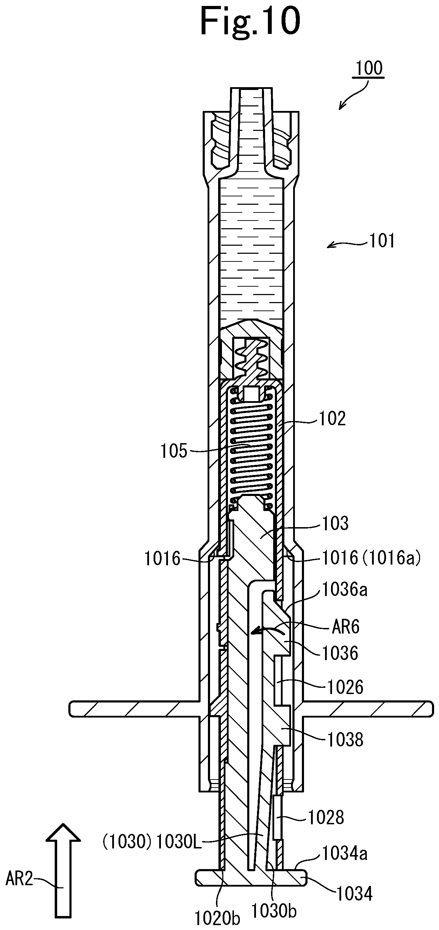

In the first administration start state (Fig. 10), the second lock part 1038 of the plunger rod 103 is locked into the first elongated hole 1026 of the plunger 102 by a biasing force of the elastic member 105. At this time, the elastic member 105 is compressed. Further, the first lock part 1036 of the plunger rod 103 projects outward in a cylinder radial direction through the first elongated hole 1026. A front end 1036a of the first lock part 1036 and a projecting part 1016a face each other. When a user press the pushing part 1034 in this state in a direction shown by an arrow AR2, another end 1020b of the plunger 102 is pushed to a surface 1034a of the pushing part 1034. Thereby, the plunger 102, the plunger rod 103, and the elastic member 105 collectively advance toward the barrel 101 to expel the medical liquid.

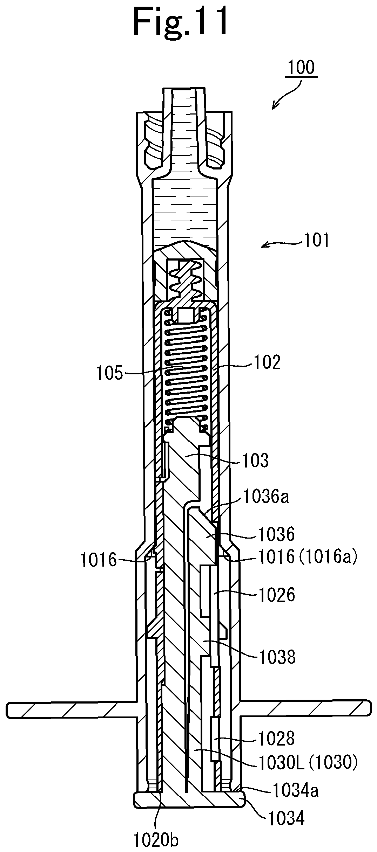

As the pushing part 1034 is pushed, the first lock part 1036 advances toward the projecting part 1016a, and then comes into contact with the projecting part 1016a. As the pushing part 1034 is further pushed, the projecting part 1016a displaces the position of the first lock part 1036. At this time, the leaf spring 1030L elastically bends toward the same direction with another end 1030b as a center. Following the displacement of the first lock part 1036, the second lock part 1038 is also displaced in the same direction to release the second lock part 1038 from lock engagement with the first elongated hole 1026. In this way, the nasal cavity administration container 100 is shifted from the first administration start state to the first administration completion state (Fig. 11).

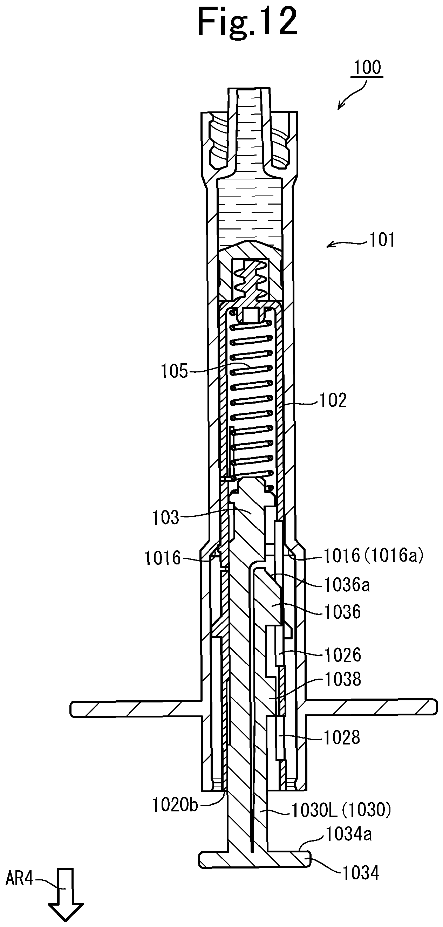

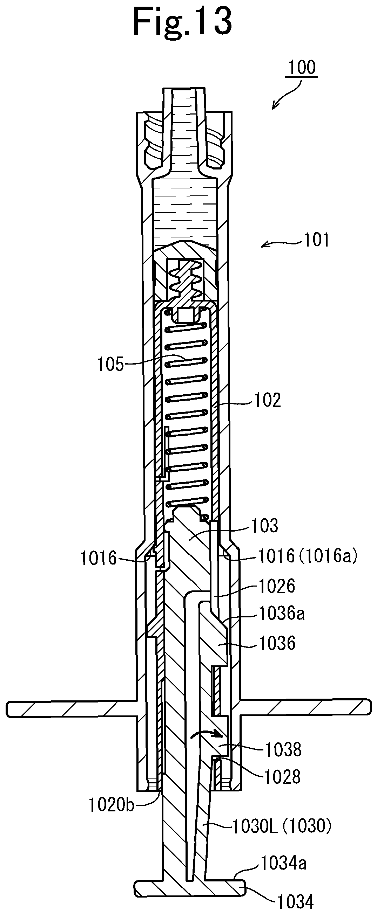

Next, when the pressure to the pushing part 1034 is released by the user, the nasal cavity administration container 100 is transferred from the first administration completion state to the pressure release state (Fig. 12). Thereby, the plunger rod 103 retracts by a restoring force of the elastic member 105 in a direction shown by an arrow AR4, and the nasal cavity administration container 100 is transferred from the pressure release state to the second administration start state (Fig. 13).

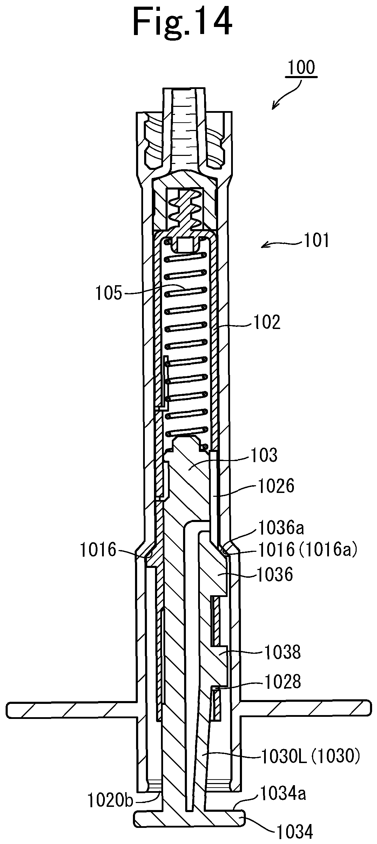

In the second administration start state, the second lock part 1038 of the plunger rod 103 is locked into the second elongated hole 1028 by the biasing force of the elastic member 105. When the user restarts the pushing to the pushing part 1034 in this state, the plunger 102, the plunger rod 103, and the elastic member 105 collectively advance toward the barrel 101 to expel the medical liquid. Thereby, the nasal cavity administration container 100 is transferred from the second administration start state to the second administration completion state (Fig. 14).

### CITATION LIST

### Patent Literature

Patent Literature 1: JP 5786852 B

### SUMMARY

### Technical Problem

In the nasal cavity administration container 100, the second lock part 1038 is locked to the first elongated hole 1026 in the first administration start state (Fig. 10), and the second lock part 1038 is locked to the second elongated hole 1028 in the second administration start state (Fig. 13). However, since the second lock part 1038 is disposed in the leaf spring 1030L as described above, the lock is released when the leaf spring 1030L is bent in this locked state. As a result, there was a possibility that the plunger 102 does not advance even by pushing the plunger rod 103, which makes it impossible to administer the medical liquid (i.e., content).

It is an object of the present invention to provide a syringe capable of dividing a content to be administered in two parts, and capable of securing the two-part administration.

### Solution to Problem

A syringe of the present invention is a syringe for dividing a content to be administered in plural parts, the syringe including: a barrel having a distal end part and a proximal end part in an axial direction and allowed to discharge the content from the distal end part; a rod configured to be inserted into the barrel from a proximal side in the axial direction and pushed to a distal side; a barrel side member attached to the barrel; a rod side member attached to the rod and configured to be shifted between a first state of being locked at a predetermined position of the rod in the axial direction and a second state of being located at a position on the proximal side in the axial direction of the first state; and a biasing member configured to be locked on the rod and the rod side member to bias the rod side member to move it from the first state toward the second state, the rod side member including: a lock part configured to be locked to the rod in the axial direction in the first state; a first pushing part configured to be pushed against the rod toward the distal side in the axial direction in the first state, and be a part separate from the lock part; and a second pushing part configured to be pushed against the rod toward the distal side in the axial direction in the second state, and be a part separate from the lock part; the rod including: a first lock part to which the lock part is locked in the first state; a first pushed part against which the first pushing part is pushed in the first state; and a second pushed part against which the second pushing part is pushed in the second state, and the barrel side member including: a lock-release part configured to release lock between the lock part and the first lock part with the rod pushed to the predetermined position in the barrel in the first state.

In the syringe, it can be configured such that the barrel includes a barrel body and a flange part disposed in the barrel body, the barrel side member is fitted onto the flange part to be attached to the barrel, the rod includes a rod body and a member attachment part disposed in the rod body, and the rod side member is attached to the member attachment part.

In the syringe, it can be configured such that the second pushing part includes: an elastic part having elasticity in a radial direction perpendicular to the axial direction; and a pushing surface part disposed in the elastic part, and having a pushing surface facing the distal side in the axial direction and being a flat surface perpendicular to the axial direction, the second pushed part includes: a pushed surface part having a pushed surface facing the proximal side in the axial direction and being a flat surface perpendicular to the axial direction, and the pushing surface is held, in the second state, by the elasticity of the elastic part at a position capable of coming into contact with the pushed surface from the proximal side in the axial direction.

### BRIEF DESCRIPTION OF DRAWINGS

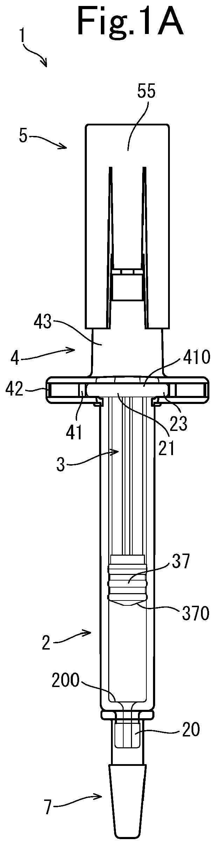

Fig. 1A is a front view of a syringe according to a first embodiment in a first state.

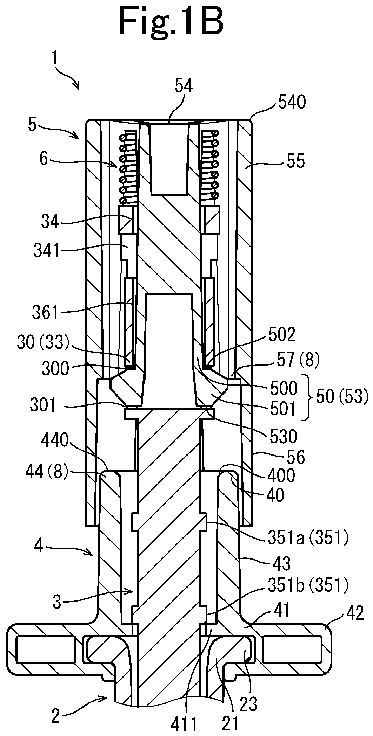

Fig. 1B is a cross-sectional view of a main part of the syringe in the first state.

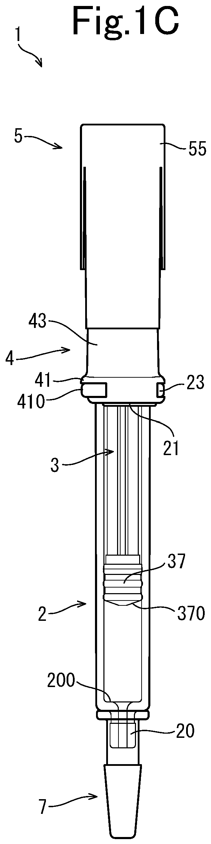

Fig. 1C is a side view of the syringe in the first state.

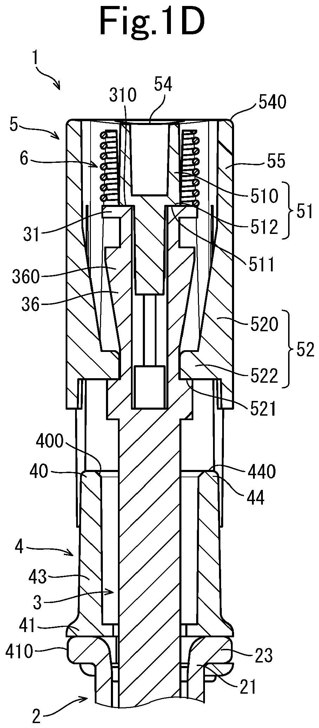

Fig. 1D is a cross-sectional view of the main part of the syringe in the first state.

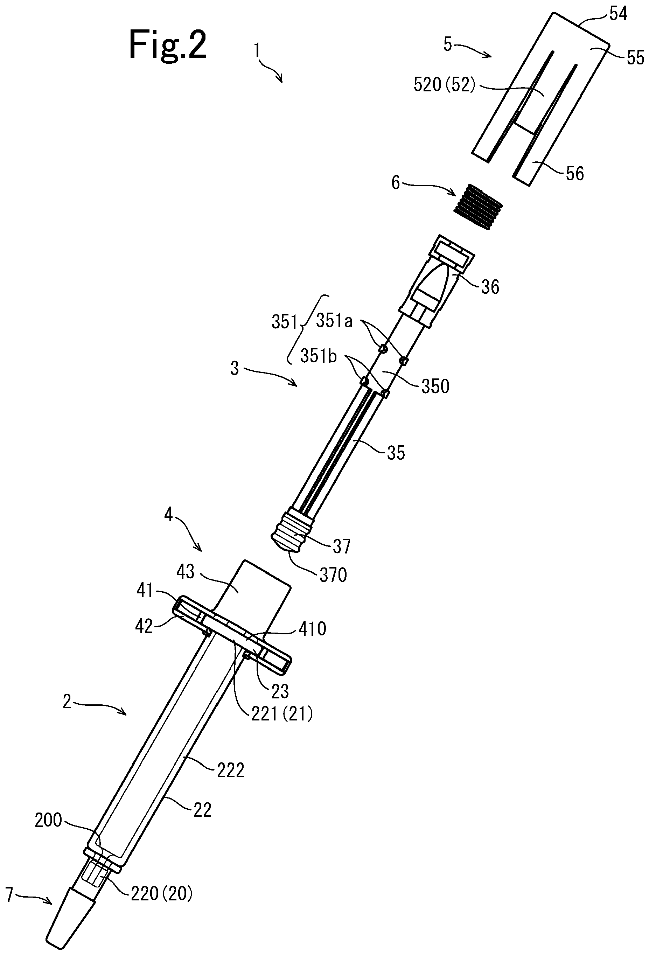

Fig. 2 is an exploded perspective view of the syringe.

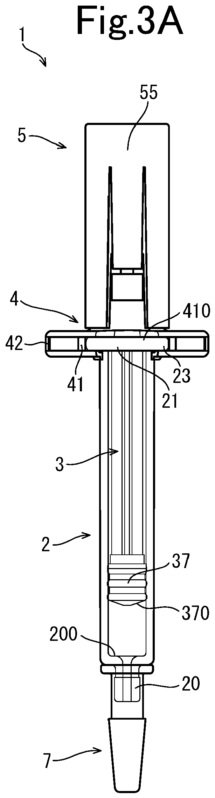

Fig. 3A is a front view of the syringe in a restricted state.

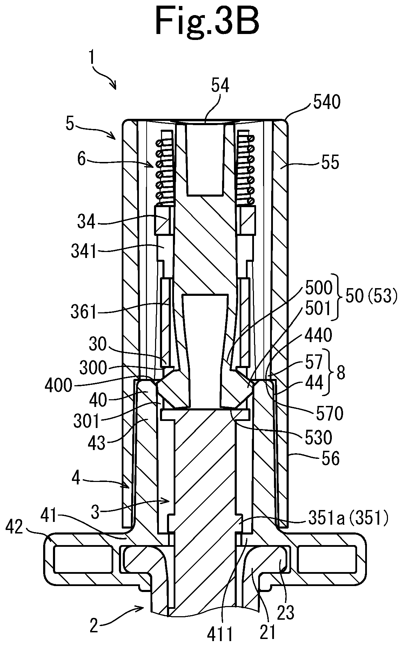

Fig. 3B is a cross-sectional view of the main part of the syringe in the restricted state.

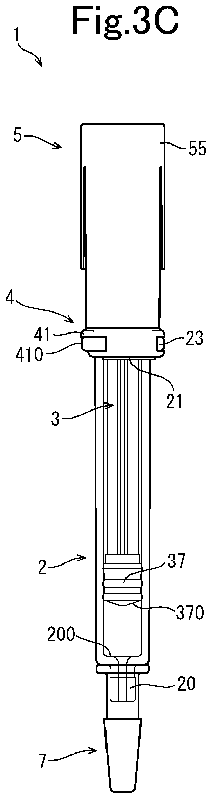

Fig. 3C is a side view of the syringe in the restricted state.

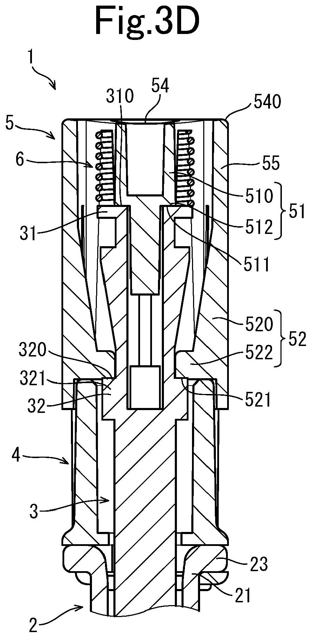

Fig. 3D is a cross-sectional view of the main part of the syringe in the restricted state.

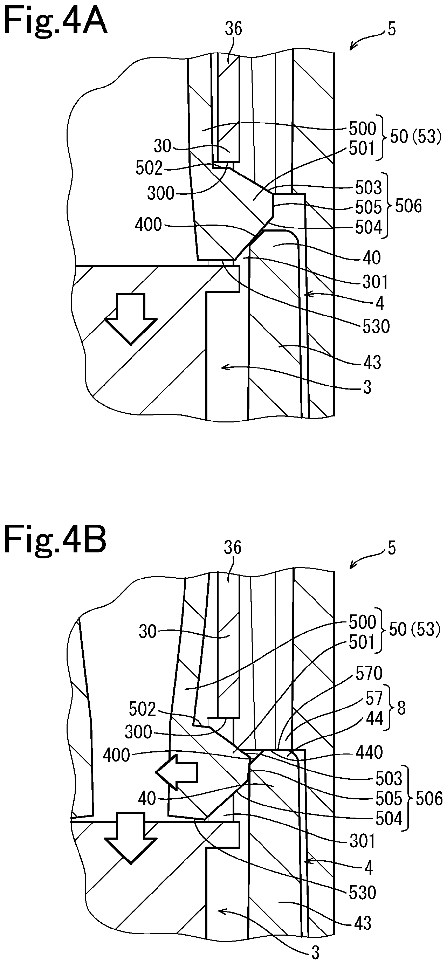

Fig. 4A is a cross-sectional view of the main part of the syringe immediately before the restricted state.
Fig. 4B is a cross-sectional view of the main part of the syringe in the restricted state.

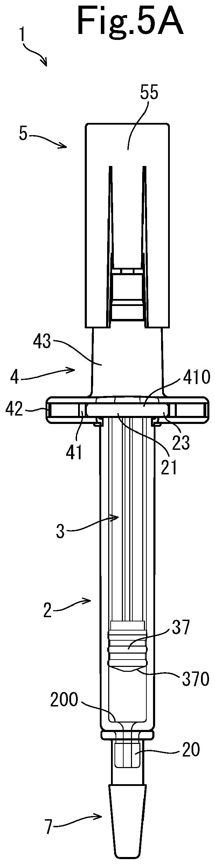

Fig. 5A is a front view of the syringe in a second state.

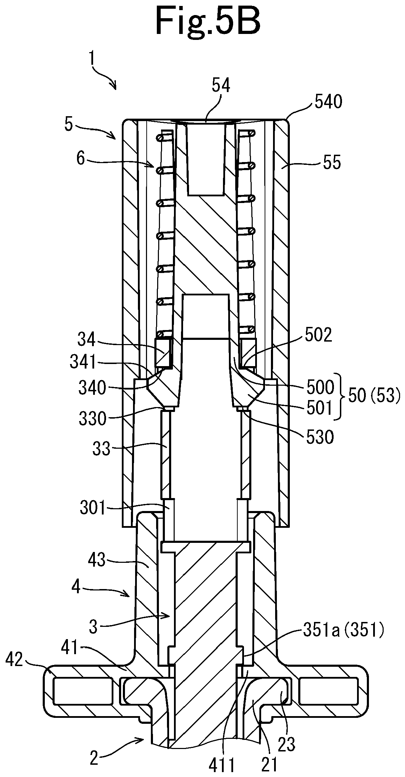

Fig. 5B is a cross-sectional view of the main part of the syringe in the second state.

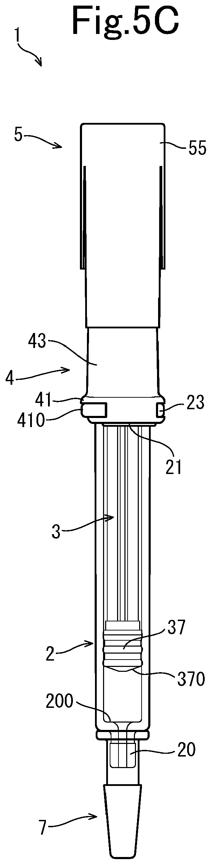

Fig. 5C is a side view of the syringe in the second state.

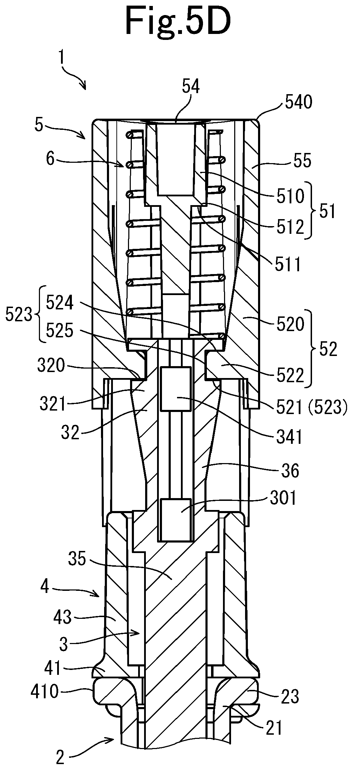

Fig. 5D is a cross-sectional view of the main part of the syringe in the second state.

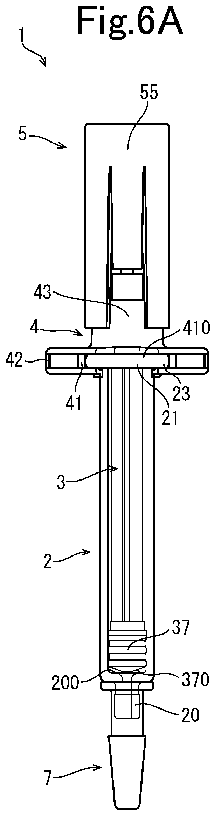

Fig. 6A is a front view of the syringe in a completion state.

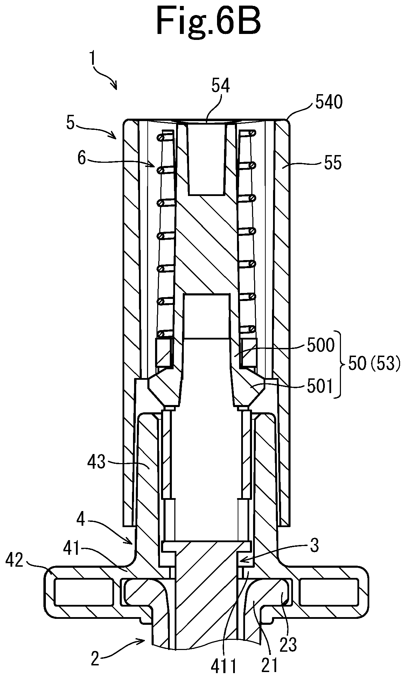

Fig. 6B is a cross-sectional view of the main part of the syringe in the completion state.

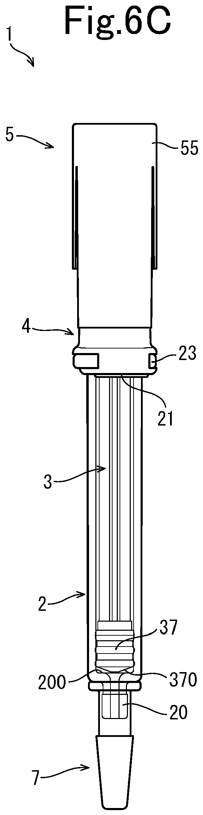

Fig. 6C is a side view of the syringe in the completion state.

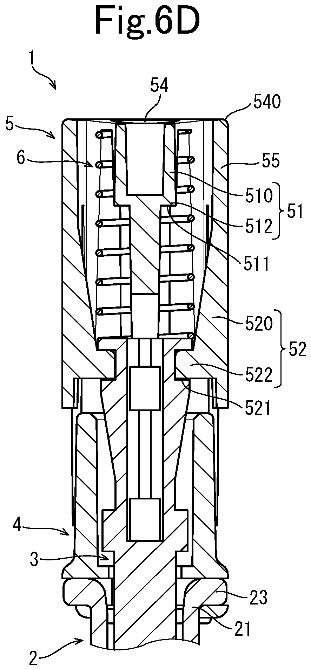

Fig. 6D is a cross-sectional view of the main part of the syringe in the completion state.

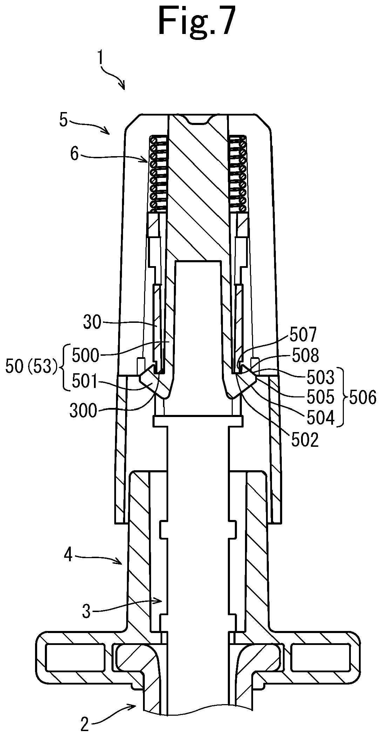

Fig. 7 is a cross-sectional view of a main part of a syringe according to a second embodiment in the first state.

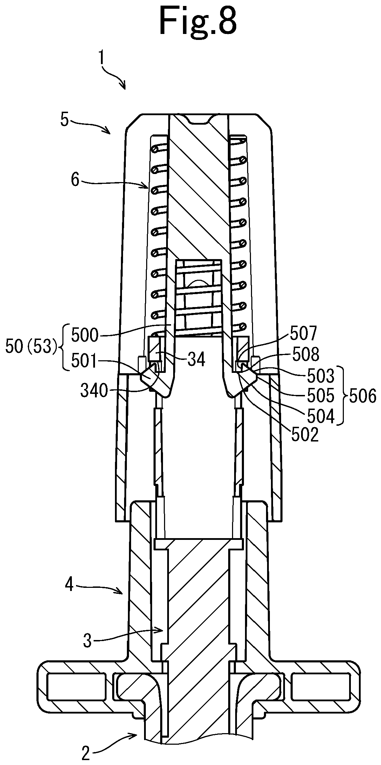

Fig. 8 is a cross-sectional view of the main part of the syringe in the second state.

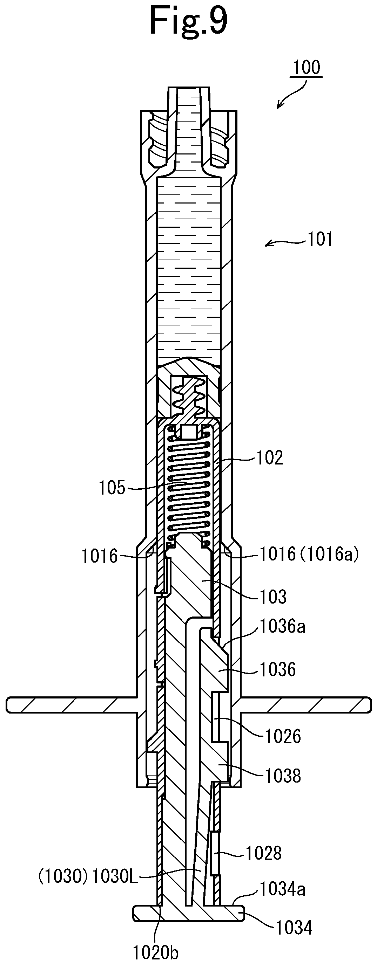

Fig. 9 is a cross-sectional view of a conventional nasal cavity administration container in a preparation state.
Fig. 10 is a cross-sectional view of the nasal cavity administration container in a first administration start state.
Fig. 11 is a cross-sectional view of the nasal cavity administration container in a first administration completion state.
Fig. 12 is a cross-sectional view of the nasal cavity administration container in a pressure release state.
Fig. 13 is a cross-sectional view of the nasal cavity administration container in a second administration start state.
Fig. 14 is a cross-sectional view of the nasal cavity administration container in a second administration completion state.

### DESCRIPTION OF EMBODIMENTS

Hereinafter, a first embodiment of the present invention will be described with reference to Fig. 1A to Fig. 6D.

As shown in Fig. 1A to Fig. 1D, a syringe 1 is a syringe for dividing a content and administering it in plural parts. Specifically, the syringe 1 is a nasal cavity administration container capable of dividing a medical liquid in a mist form to administer the same into both nostrils. According to this syringe 1, the medical liquid is divided to be administered in two parts, but the medical liquid can be divided to be administered in three or more parts, or a plurality of parts. The medical liquid is a medical liquid in a liquid form and, for example, an influenza vaccine. The syringe 1 has a substantially cylindrical shape.

As shown in Fig. 2, the syringe 1 has a distal end part 20 and a proximal end part 21 in an axial direction and includes a barrel 2 capable of discharging a content from the distal end part 20, and a rod 3 to be inserted into the barrel 2 from the proximal side in the axial direction and pushed toward the distal end. The syringe 1 includes a barrel side member 4 attached to the barrel 2 and a rod side member 5 attached to the rod 3 and configured to be movable between a first state of being locked at a predetermined position of the rod 3 in the axial direction and a second state of being located at a position on the proximal side in the axial direction of the first state. The syringe 1 further includes a biasing member 6 configured to be locked on the rod 3 and the rod side member 5, to bias the rod side member 5 to move it from the first state toward the second state.

The syringe 1 of this embodiment further includes an injection nozzle 7. The syringe 1 further includes a pushing-restriction mechanism 8 that restricts the rod side member 5 from being pushed toward the distal end after the content is first discharged and before the rod 3 shifts into the second state (see Fig. 3A and Fig. 3B).

In the syringe 1, a side where the distal end part 20 of the barrel 2 (i.e., lower side in Fig. 1A to Fig. 1D and Fig. 3A to Fig. 6D) is disposed is referred to as "distal side", and a side where the proximal end part 21 of the barrel 2 (i.e., upper side in Fig. 1A to Fig. 1D and Fig. 3A to Fig. 6D) is disposed is referred to as "proximal side". The "axial direction" of the syringe 1 refers to a cylinder axis direction of the syringe 1. A radial direction of the syringe 1 may be also simply referred to as "radial direction".

When using the syringe 1, the rod 3 in the first state where the rod 3 is raised on the proximal side (see Fig. 1A to Fig. 1D) is pushed to the distal side to perform a first spraying of the medical liquid, and then brought into a restricted state (see Fig. 3A to Fig. 3D). Next, the rod 3 is transferred through a second state where the rod 3 is raised on the proximal side (see Fig. 5A to Fig. 5D), further pushed to perform a second spraying of the medical liquid, and then brought into a complete state (see Fig. 6A to Fig. 6D).

Hereinafter, the structures of the syringe 1 will be described in details.

The barrel 2 is a member having an inside for containing a medical liquid (see Fig. 2). In the syringe 1 of this embodiment, the barrel 2 includes a barrel body 22 and a flange part 23 disposed on the barrel body 22.

The barrel body 22 is a cylindrical part. In this barrel 2, a body distal end part 220 being a distal end part of the barrel body 22 forms the distal end part 20 of the barrel 2, and a body proximal end part 221 being a proximal end part of the barrel body 22 forms the proximal end part 21 of the barrel 2. In addition to the body distal end part 220 and the body proximal end part 221, the barrel body 22 includes a body connection part 222 having a cylindrical shape and connecting the body distal end part 220 and the body proximal end part 221.

The flange part 23 extends outward (i.e., radially outward direction of the barrel body 22) from, for example, an entire outer circumference on one end in the cylinder axis direction of the proximal end part 21. That is, the flange part 23 has a ring shape (e.g., annular shape) when viewed from the cylinder axis direction.

An outer diameter of the body distal end part 220 is smaller than that of the body connection part 222. The outer diameter of the body connection part 222 is substantially constant at any portion in the cylinder axis direction.

The barrel 2 is made of, for example, a material that is transparent and can withstand the internal pressure applied thereto at the time of administration of the medical liquid. Specifically, the material of the barrel 2 is a resin having a cyclic olefin such as norbornene in a repeating unit, glass, or the like. More specifically, the material of the barrel 2 is a transparent resin such as a COP (cycloolefin polymer) which is a homopolymer of a cyclic olefin, or a COC (cycloolefin copolymer) which is a copolymer of a cyclic olefin and ethylene or the like, glass, or the like.

The rod side member 5 is a member to be operated at the time of discharging the content in the barrel 2. An appearance of the rod side member 5 is, for example, substantially cylindrical shape. The rod side member 5 includes a lock part 50, a first pushing part 51, and a second pushing part 52 (see Fig. 1A to Fig. 1D). In the syringe 1 of this embodiment, the rod side member 5 includes a third pushing part 53 that can be pushed in the second state toward the distal end of the rod in the axial direction.

The rod side member 5 further includes a top panel part 54 and a skirt part 55 extending from the top panel part 54 to the distal side. The rod side member 5 further includes an extending part 56 that covers the lock part 50 from the radially outside. The rod side member 5 includes a push contact part 57 that forms a part of the push restriction mechanism 8.

The top panel part 54 has a flat plate shape along in the radial direction, and has a disc shape in this embodiment. Further, the top panel part 54 is pushed to the distal side by a user of the syringe 1 at the time of discharging the content in the barrel 2. An outer circumferential portion 540 of the top panel part 54 is formed in a round chamfer, but can be formed in a C chamfer.

The skirt part 55 extends from the outer circumferential portion 540 of the top panel part 54 to the distal side. The skirt part 55 is continuous throughout the entire outer circumference of the top panel part 54 and has, for example, a cylindrical shape.

The lock part 50 is locked to the rod 3 in the axial direction in the first state (see Fig. 1A and Fig. 1B). A pair of lock parts 50 are disposed to be opposed to each other in the radial direction with the axis of the rod 3 interposed therebetween. The pair of lock parts 50 are, for example, arranged to be located on the opposite sides with the rod 3 interposed therebetween. Further, the lock parts 50 extend from an inner portion of the outer circumferential portion 540 of the top panel part 54 to the distal side. For example, the lock parts 50 each include a lock elastic part 500 having elasticity in the radial direction orthogonal to the axial direction, and a lock piece 501 disposed in the lock elastic part 500 and extending radially outward.

The lock elastic part 500 is a plate-shaped (e.g., elongated plate-shaped) member extending in the axial direction. The lock elastic part 500 extends from the inner portion of the outer circumferential portion 540 of the top panel part 54 to the distal side.

The lock piece 501 extends radially outward from the distal end part of the lock elastic part 500. As shown in Fig. 4A and Fig. 4B, the lock piece 501 has a lock surface 502 facing the proximal side in the axial direction and being a flat surface perpendicular to the axial direction. Further, an outer surface 506 that faces the outside in the radial direction of the lock piece 501 has a first outer surface 503 facing the proximal side and a second outer surface 504 facing the distal side. The outer surface 506 further has a third outer surface 505 connecting the first outer surface 503 and the second outer surface 504.

The first outer surface 503 extends to incline relative to the radial direction orthogonal to the axial direction. The first outer surface 503 inclines to be located radially inward as it advances to the proximal side. In this lock piece 501, the first outer surface 503 extends from the radially outward end of the lock surface 502. The first outer surface 503 is located on the proximal side in the axial direction relative to the third outer surface 505.

The second outer surface 504 extends to incline relative to the radial direction orthogonal to the axial direction. The second outer surface 504 inclines to be located radially outward as it advances to the proximal side. Further, the second outer surface 504 is located on the distal side in the axial direction relative to the third outer surface 505.

The third outer surface 505 extends along the axial direction. The third outer surface 505 is located on the distal side in the axial direction relative to the first outer surface 503.

The first pushing part 51 extends, for example, from the top panel part 54 (specifically, the radially inward portion of the outer circumferential portion 540 of the top panel 54) to the distal side (see Fig. 1C and Fig. 1D). In the first state, the first pushing part 51 can be pushed to the distal side in the axial direction relative to the rod 3. The first pushing part 51 is a different part from the lock part 50.

In the syringe 1 of this embodiment, the first pushing part 51 includes an extending part 510 extending from the top panel part 54 to the distal side, and a first pushing surface part 512 having a first pushing surface 511 facing the distal side in the axial direction and being a flat surface perpendicular to the axial direction.

The second pushing part 52 extends, for example, from the skirt part 55 to the distal side, as shown in Fig. 5C and Fig. 5D. A pair of second pushing parts 52 are disposed in the radial direction with the axis of the rod 3 interposed therebetween. The second pushing parts 52 are arranged between the pair of lock parts 50 in the circumferential direction. Specifically, the second pushing parts 52 are arranged between the pair of lock parts 50 at equal intervals from the lock parts 50 in the circumferential direction. That is, the rod side member 5 of this embodiment includes a plurality of lock parts 50 disposed at equal intervals in the circumferential direction, and a plurality of second pushing parts 52 arranged between the lock parts 50 that are adjacent to each other in the circumferential direction. In the second state, the second pushing part 52 can be pushed to the distal side in the axial direction relative to the rod 3. The second pushing part 52 is a different part from the lock part 50. The second pushing part 52 is formed to extend radially inward from the radially outside.

In the syringe 1 of this embodiment, the second pushing part 52 includes an elastic part 520 having elasticity in the radial direction orthogonal to the axial direction. The second pushing part 52 further includes a second pushing surface part 522 as a pushing surface part that is disposed in the elastic part 520 and has a second pushing surface 521 serving as a pushing surface, in which the second pushing surface 521 faces the distal side in the axial direction and is a flat surface perpendicular to the axial direction.

The elastic part 520 is a member having a plate shape (e.g., elongated plate shape) extending along the axial direction. The elastic part 520 extends from the outer circumferential portion 540 of the top panel part 54 to the distal side.

The second pushing surface 521 is a flat surface disposed to extend radially inward from an inner circumference of the rod side member 5.

The second pushing surface part 522 extends radially inward from the distal end part of the elastic part 520. An inner circumferential surface 523 of the second pushing surface part 522 has, in addition to the second pushing surface 521, a proximal side inner circumferential surface 524 facing the proximal side in the axial direction and being a flat surface perpendicular to the axial direction. The inner circumferential surface 523 has a connecting inner circumferential surface 525 extending along the axial direction and connecting the second pushing surface 521 and the proximal side inner circumferential surface 524.

As shown in Fig. 5A and Fig. 5B, the third pushing part 53 extends, for example, from the top panel part 54 (specifically, the radially inward portion of the outer circumferential portion 540 of the top panel part 54) to the distal side. The third pushing part 53 has a third pushing surface 530 facing the distal side in the axial direction and being a flat surface perpendicular to the axial direction.

In the syringe 1 of this embodiment, the lock part 50 also serves as the third pushing part 53, but the third pushing part 53 can be a different part from the lock part 50 in the rod side member 5.

As shown in Fig. 3A and Fig. 4B, the push contact part 57 that forms the push restriction mechanism 8 extends from the top panel part 54 (specifically, the outer circumferential portion 540 of the top panel part 54) to the distal side. The push contact part 57 has a push contact surface 570 facing the distal side in the axial direction and being a flat surface perpendicular to the axial direction.

In the syringe 1 of this embodiment, the push contact surface 570 is disposed partially in the circumferential direction, but can be disposed continuously throughout the entire circumference.

The rod 3 is a member that moves along with the rod side member 5 at the time of discharging the content in the barrel 2. The rod 3 has a shaft shape.

The rod 3 includes a first lock part 30, a first pushed part 31, and a second pushed part 32 (see Fig. 1A to Fig. 1D and Fig. 3C, and Fig. 3D). The rod 3 further includes a third pushed part 33. The rod 3 further includes a second lock part 34 (see Fig. 5A and Fig. 5B).

In the syringe 1 of this embodiment, the rod 3 includes a rod body 35, and a member attachment part 36 disposed in the rod body 35 (see Fig. 2). The rod 3 further includes a piston 37 disposed on the rod body 35. In the rod 3, the member attachment part 36 is disposed at a proximal end part of the rod body 35, and the piston 37 is disposed at a distal end part of the rod body 35. The rod 3 is inserted into the barrel 2 with the member attachment part 36 located on the proximal side and the piston 37 located on the distal side.

In this rod 3, the member attachment part 36, the rod body 35, and the piston 37 are continuous from the proximal side to the distal side in the axial direction. In this rod 3, the rod body 35 and the member attachment part 36 are formed integrally. The rod body 35 and the piston 37 are separate members and these members are connected to form the rod 3. At least two out of the rod body 35, the member attachment part 36, and the piston 37 can be formed integrally, or all of them can be separate members.

The rod body 35 is a portion in a shaft shape. The rod 3 has a shaft part 350, and a plurality of (e.g., four) pressure accumulating projections 351 projecting from the shaft part 350 radially outward of the rod 3. Specifically, the pressure accumulating projections 351 include a plurality of (e.g., two) proximal pressure accumulating projections 351a disposed on the proximal side, and a plurality of (e.g., two) distal pressure accumulating projections 351b. In this rod 3, the plurality of proximal pressure accumulating projections 351a are disposed at equal intervals in the circumferential direction of the rod 3. In the same manner, the distal pressure accumulating projections 351b are disposed at equal intervals in the circumferential direction of the rod 3.

The rod side member 5 is attached to the member attachment part 36. The member attachment part 36 has, for example, a widened part 360 having a width in side view increased as it advances to the proximal side. Insertion holes 361 extending along the axial direction are formed at a center in the radial direction of the member attachment part 36 (see Fig. 1A). The pair of lock parts 50 of the rod side member 5 are respectively inserted into these insertion holes 361.

The piston 37 is a member that can come into contact with an inner surface 200 located on the proximal side of the distal end part 20 of the barrel 2. The piston 37 comes into close contact with the entire circumference of the inner surface of the body connection part 222 of the barrel 2. As shown in Fig. 6A to Fig. 6D, a distal end surface 370 of the piston 37 of this embodiment comes into contact with the inner surface 200 of the distal end part 20 of the barrel 2 at the time of completion of administration of the medical liquid (specifically, after the second discharge of the medical liquid).

In the first state, the first lock part 30 is locked to the lock part 50 of the rod side member 5 (see Fig. 1A and Fig. 1B). The first lock part 30 has a first lock surface 300 facing the distal side in the axial direction and being a flat surface perpendicular to the axial direction. This first engaging surface 300 is locked to the lock surface 502 (see Fig. 4A). That is, the lock part 50 and the first lock part 30 are locked to each other in the axial direction in the position where the lock part 50 is located on the distal side in the axial direction and the first lock part 30 is located on the proximal side in the axial direction. With this locking, the rod side member 5 located at a position in the first state is restricted from moving to the proximal side in the axial direction (i.e., direction in which the rod side member 5 shifts into the second state) relative to the rod 3.

A pair of first lock parts 30 are disposed to be opposed to each other in the radial direction with the axis of the rod 3 interposed therebetween. The pair of lock parts 30 are, for example, arranged to be located on the opposite sides with the rod 3 interposed therebetween. In the syringe 1 of this embodiment, the first lock part 30 is a distal end part of the member attachment part 36. The first lock part 30 is a through-hole part having a first through-hole 301 formed to pass through in the radial direction.

Further, in the syringe 1 of this embodiment, the extending part 56 covers the lock part 50 from the radially outside in the rod side member 5. Therefore, it is possible to prevent the user when in use of the syringe 1 from touching the lock part 50 and prevent the user from releasing the lock between the lock part 50 and the first lock part 30 unintentionally.

The second lock part 34 is locked to the lock part 50 of the rod side member 5 in the second state. The second lock part 34 has a second lock surface 340 facing the distal side in the axial direction and being a flat surface perpendicular to the axial direction. A pair of second lock parts 34 are disposed in the radial direction with the axis of the rod 3 interposed therebetween. The second lock part 34 is through-hole part having a second through-hole 341 formed to pass through in the radial direction.

The first pushing part 51 of the rod side member 5 is pushed against the first pushed part 31,in the first state (see Fig. 1C and Fig. 1D). The first pushed part 31 has a first pushed surface 310 facing the proximal side in the axial direction and being a flat surface perpendicular to the axial direction. This first pushed surface 310 is pushed against the first pushing surface 511. In the syringe 1 of this embodiment, the first pushed part 31 is a proximal end part of the rod 3 (specifically, the proximal end part of the member attachment part 36).

The second pushing part 52 of the rod side member 5 is pushed against the second pushed part 32 in the second state (see Fig. 5C and Fig. 5D). The second pushed part 32 further includes a second pushed surface part 321 as a pushed surface part that has a second pushed surface 320 facing the proximal side in the axial direction and serving as a pushed surface being a flat surface perpendicular to the axial direction. The second pushed part 32 is formed radially outward from the radially inside of the rod 3.

Specifically, the second pushed surface 320 is a flat surface extending radially outward from the outer circumference of the member attachment part 36. In the syringe 1 of this embodiment, the second pushed part 32 is disposed on the proximal end part of the member attachment part 36. The second pushed part 32 is recessed in the radially inward direction and formed to be stepwise in the second pushed surface 320.

A pair of second pushed parts 32 are disposed in the radial direction with the axis of the rod 3 interposed therebetween. The second pushed parts 32 are arranged between the pair of second lock parts 34 in the circumferential direction. Specifically, the second pushed parts 32 are arranged between the pair of second lock parts 34 at equal intervals from the second lock parts 34 in the circumferential direction. That is, the rod 3 of this embodiment includes a plurality of second lock parts 34 disposed at equal intervals in the circumferential direction, and a plurality of second pushed parts 32 arranged between the second lock parts 34 that are adjacent to each other in the circumferential direction.

The third pushing part 53 of the rod side member 5 is pushed against the third pushed part 33 in the second state (see Fig. 5A and Fig. 5B). The third pushed part 33 has a third pushed surface 330 facing the proximal side in the axial direction and being a flat surface perpendicular to the axial direction. The third pushed part 33 and the second lock part 34 are disposed to be opposed to each other in the axial direction. In this syringe 1, the third pushed part 33 is disposed on the distal side in the axial direction, and the second lock part 34 is disposed on the proximal side in the axial direction. In the second state, the lock part 50 is positioned between the third pushed part 33 and the second lock part 34.

Further, a pair of third pushed parts 33 are disposed with the axis of the rod 3 interposed therebetween. The third pushed parts 33 are arranged between the pair of second pushed parts 32 in the circumferential direction. Specifically, the third pushed parts 33 arranged between the pair of second pushed parts 32 at equal intervals from the second pushed parts 32 in the circumferential direction. That is, the rod 3 of this embodiment includes a plurality of second pushed parts 32 disposed at equal intervals in the circumferential direction, and a plurality of third pushed parts 33 disposed between the second pushed part 32 that are adjacent to each other in the circumferential direction.

Note that a hole forming surface on the proximal side of the second through-hole 341 in the through-hole part is the second lock surface 340, and a hole forming surface on the distal side of the second through-hole 341 is the third pushed surface 330.

In this syringe 1, the lock part 50 is fitted to the first lock part 30 from the radially inside to allow the first lock part 30 and the lock part 50 to be locked with each other (i.e., first state, Fig. 1A and Fig. 1B). When the lock part 50 is retracted radially inward from the first lock part 30, the locking is released (Fig. 4A and Fig. 4B). Thereby, the lock part 50 shifts the insertion hole 361 to the proximal side to be fitted to the second lock part 34 from the radially inside. Thereby, the second lock part 34 and the lock part 50 are locked to each other (i.e., second state, Fig. 5A and Fig. 5B).

The material of the rod 3 is a resin such as POM (polyacetal).

The barrel side member 4 includes a lock-release part 40 that releases the lock between the lock part 50 of the rod side member 5 and the first lock part 30 of the rod 3 in the state where the rod 3 is pushed to a predetermined position in the barrel 2 in the first state (see Fig. 1A and Fig. 1B). In the syringe 1 of this embodiment, the barrel side member 4 is fitted onto the flange part 23 of the barrel 2 to be attached to the barrel 2.

The barrel side member 4 includes, in addition to the lock-release part 40, a barrel attachment part 41 being a portion to be fitted onto the flange part 23 of the barrel 2, and a finger hook part 42 functioning as a finger grip. With such a configuration in which the lock-release part 40, the barrel attachment part 41, and the finger hook part 42 are formed by the barrel side member 4 as a single member, the structure of the syringe 1 can be simplified.

The barrel side member 4 further includes a barrel side body 43 having a cylindrical shape. In this barrel side member 4, the barrel side body 43 and the barrel attachment part 41 are continuous from the proximal side to the distal side. The barrel side member 4 further includes a push restriction part 44 that forms a part of the push restriction mechanism 8.

The barrel attachment part 41 extends continuously in the circumference direction of the barrel 2. That is, the barrel attachment part 41 is formed into a ring shape. The barrel attachment part 41 is provided with an attachment part through-hole 410 through which the flange part 23 of the barrel 2 is partly exposed. A pair of attachment part through-holes 410 are disposed in the barrel attachment part 41. In the syringe 1 of this embodiment, the barrel attachment part 41 includes a pressure accumulating part 411 that extends radially inward and can be locked to the pressure accumulating projection 351.

The finger hook part 42 extends radially outward of the barrel attachment part 41. For example, a pair of finger hook parts 42 are disposed with the barrel 2 interposed therebetween. The finger hook part 42 is disposed in the barrel attachment part 41 at a position avoiding the attachment part through-hole 410.

The barrel side body 43 is a portion located on the proximal side of the barrel 2. The barrel side body 43 extends from the proximal end part further to the proximal side of the barrel attachment part 41. Further, the barrel side body 43 has, for example, a cylindrical shape. The barrel side body 43 has an outer diameter substantially equal to any portion in the axial direction, or has a tapered shape tapering toward the distal side in the axial direction.

The lock-release part 40 is a portion configured to release the lock between the lock part 50 of the rod side member 5 and the first lock part 30 of the rod 3 in the state where the rod 3 is pushed to a predetermined position of the barrel 2 in the first state (see Fig. 4A and Fig. 4B). The lock-release part 40 has, for example, an inclined surface 400 facing the proximal side and inclined to the radial direction orthogonal to the axial direction. In the syringe 1 of this embodiment, the lock-release part 40 is located on the proximal end part of the barrel side body 43.

The push restriction part 44 forming the push restriction mechanism 8 is a portion configured to restrict the push contact part 57 of the rod side member 5 from moving to the distal side. The push restriction part 44 has, for example, a push restriction surface 440 facing the proximal side and being a flat surface perpendicular to the radial direction orthogonal to the axial direction. The push contact surface 570 comes into contact with the push restriction surface 440. In the syringe 1 of this embodiment, the push restriction part 44 is located on the distal end part of the barrel side body 43.

In the syringe 1 of this embodiment, the push restriction surface 440 is disposed continuously throughout the entire circumference, but can be disposed partially in the circumferential direction.

The material of the barrel side member 4 is a resin such as PP (polypropylene).

The biasing member 6 is an elastic member disposed, together with the proximal end part of the rod 3 (e.g., member attachment part 36), inside the rod side member 5. This biasing member 6 is arranged between the top panel part 54 of the rod side member 5 and the member attachment part 36. The biasing member 6 is configured to be locked on the top panel part 54 and the member attachment part 36, to bias the rod side member 5 to move it to the proximal side (i.e., first state).

In the syringe 1 of this embodiment, the biasing member 6 is, for example, a spring (specifically, metal spring) (see Fig. 2). Specifically, the biasing member 6 is a compression spring.

The biasing member 6 can be, for example, a member other than the metal spring, such as a resin spring. In the case where the syringe 1 includes a resin spring as the biasing member 6, the resin spring and the rod 3 can be integrally molded, or the resin spring and the rod side member 5 can be integrally molded.

The injection nozzle 7 is a portion configured to extract the medical liquid. The injection nozzle 7 of this embodiment is configured to expel the medical liquid in the form of mist.

Hereinafter, the detailed description will be given on the way of operation and the respective states when the syringe 1 is used.

The first state is an initial state in which the medical liquid has not yet been expelled. In the first state, the lock part 50 is locked on the first lock part 30 of the rod 3, to thereby lock the rod side member 5 on the rod 3 at a predetermined position in the axial direction (see Fig. 1A and Fig. 1B). Specifically, the lock surface 502 of the lock part 50 is locked on the first lock surface 300 of the first lock part 30, to thereby lock the rod side member 5 on the rod 3 at a predetermined position in the axial direction. The lock of the lock part 50 and the first lock part 30 restricts the rod side member 5 from moving to the proximal side.

When the user of the syringe 1 pushes the rod side member 5 (specifically, the top panel part 54) from the first state to the distal side (see Fig. 1C and Fig. 1D), the first pushing part 51 of the rod side member 5 is pushed to the first pushed part 31 of the rod 3. Thereby, the rod 3 and the rod side member 5 are pushed to the distal side to cause the first medical liquid spray to move into the restricted state (see Fig. 3C and Fig. 3D). Specifically, the first pushing surface 511 of the first pushing part 51 is pushed to the first pushed surface 310 of the first pushed part 31.

From the first state to the restricted state, the lock of the lock part 50 and the first lock part 30 allows the biasing member 6 to be maintained in the state of biasing the rod side member 5 to move it toward the second state.

In the syringe 1 of this embodiment, a pressure accumulating means is formed by the pressure accumulating part 411 of the barrel side member 4 and the pressure accumulating projection 351 of the rod 3 in the first medical liquid spray (see Fig. 1A and Fig. 1B). Specifically, the rod 3 receives a resistive force in the first medical liquid spray when the distal pressure accumulating projections 351b of the rod 3 get over or ride over the pressure accumulating part 411 upon pushing of the rod 3 together with the rod side member 5. Thereby, the rod 3 can be biased to such a level as that the medical liquid can be discharged in the mist form in the first medical liquid spray.

The syringe 1 shifts into the restricted state upon the first medical liquid spray performed by pushing the rod 3 to the distal side as described above (see Fig. 3A to Fig. 3D). The first medical liquid spray is completed at the position where the rod side member 5 is in contact with the barrel side member 4. For example, the first medical liquid spray is completed by the operation of the push restriction mechanism 8. Specifically, the first medical liquid spray is completed when the push contact part 57 of the rod side member 5 comes into contact with the push restriction part 44 of the barrel side member 4. At this time, in the syringe 1 of this embodiment, the push contact surface 570 of the push contact part 57 comes into contact with the push restriction surface 440 of the push restriction part 44 (see Fig. 3A and Fig. 3B).

As described above, the syringe 1 of this embodiment includes the push restriction mechanism 8 that restricts, when the rod 3 and the rod side member 5 are pushed from the first state by a predetermined length (for the first injection), the rod side member 5 from being pushed to the distal side. This configuration can prevent unnecessary pushing of the rod 3 to the distal side in the first administration and allows an appropriate amount of content to be discharged in the first administration.

When the first medical liquid spray is completed, the lock-release part 40 of the barrel side member 4 releases the lock between the lock part 50 of the rod side member 5 and the first lock part 30 of the rod 3 (see Fig. 4A and Fig. 4B). Specifically, since the second outer surface 504 of the lock part 50 is guided along the inclined surface 400 of the lock-release part 40, the lock elastic part 500 of the lock part 50 is bent and deformed radially inward, to allow the lock part 50 to be entirely moved radially inward. Thereby, the lock between the lock part 50 and the first lock part 30 (in the syringe 1 of this embodiment, the lock between the lock surface 502 of the lock part 50 and the first lock surface 300 of the first lock part 30) is released. That is, the second outer surface 504 functions as a release guide part to allow the lock-release part 40 to guide the lock part 50 to release the lock.

When the lock of the lock part 50 to the first lock part 30 is released, the rod side member 5 starts to move to the proximal side by the biasing force of the biasing member 6. When the lock part 50 has moved to the proximal side, the first outer surface 503 comes into contact with the first lock surface 300 of the first lock part 30. The first outer surface 503, which is an inclined surface inclined to be located radially inside as it advances to the proximal side, enhances the lock part 50 to be retracted radially inward. That is, the first outer surface 503 functions as a release enhancement part to enhance release of the lock of the lock part 50 to the first lock part 30.

When the lock between the lock part 50 and the first lock part 30 is released from the restricted state, the biasing member 6 is released from the compression in the first state to move the rod side member 5 from the first state to the second state, thereby shifting into the second state (see Fig. 5A to Fig. 5D). At this time, upon the release from the compression in the first state, the biasing member 6 allows the rod side member 5 to move from the distal side to the proximal side relative to the rod 3, thereby shifting into the second state. That is, the rod side member 5 is moved by a restoring force of a spring that is the biasing member 6, thereby shifting into the second state. In the syringe 1 of this embodiment, the rod side member 5 stops at a position where the lock part 50 of the rod side member 5 is locked to the second lock part 34 of the rod 3 (specifically, the second lock surface 340).

The second state is an intermediate state where the injection subsequent to the first medical liquid injection is prepared. In the second state, the second pushing part 52 of the rod side member 5 can be pushed to the distal side in the axial direction relative to the rod 3 (see Fig. 5C and Fig. 5D). In this state, the second pushing surface 521 of the second pushing part 52 is held, by the elasticity of the elastic part 520 of the second pushing part 52, at a position capable of coming into contact with the second pushed surface 320 of the second pushed part 32 from the proximal side in the axial direction.

Further, in the second state, the lock part 50 of the rod side member 5 can be pushed toward the proximal side in the axial direction relative to the rod 3 (see Fig. 5A and Fig. 5B). In this state, the lock surface 502 of the lock part 50 is held, by the elasticity of the lock elastic part 500 of the lock part 50, at a position capable of coming into contact with the second lock surface 340 of the second lock part 34 from the distal side in the axial direction.

When the user of the syringe 1 pushes the rod side member 5 (specifically, the top panel part 54) from the second state to the distal side, the second pushing part 52 of the rod side member 5 is pushed to the second pushed part 32 of the rod 3 and push the rod 3 to the distal side together with the rod side member 5, to cause the second medical liquid spray, thereby shifting into the completion state (see Fig. 6A to Fig. 6D). Specifically, the second pushing surface 521 of the second pushing part 52 is pushed to the second pushed surface 320 of the second pushed part 32.

In the syringe 1 of this embodiment, in the second state, the third pushing part 53 of the rod side member 5 is pushed to the third pushed part 33 of the rod 3. Thus, when the rod side member 5 is pushed in order to carry out the pushing of the rod 3 to the barrel 2 in the second state, the third pushing part 53 is pushed against the third pushed part 33, while the second pushing part 52 of the rod side member 5 is pushed against the second pushed part 32, to allow the rod 3 to move securely to the distal side in the axial direction. Thereby, the second administration can be performed.

Further, the lock part 50 of the rod side member 5 also serving as the third pushing part 53 enables a simplified structure of the syringe 1.

In the second medical liquid spray, the pressure accumulating means is formed by the pressure accumulating part 411 of the barrel side member 4 and the pressure accumulating projection 351 of the rod 3 (see Fig. 5A and Fig. 5B). Specifically, the rod 3 receives a resistive force in the second medical liquid spray when the proximal pressure accumulating projections 351a of the rod 3 get over or ride over the pressure accumulating part 411 upon pushing of the rod 3 together with the rod side member 5. Thereby, the rod 3 can be biased to such a level as that the medical liquid can be discharged in the mist form in the second medical liquid spray.

The syringe 1 shifts into the completion state upon the performance of the second medical liquid spray by pushing the rod 3 to the distal side in this manner (see Fig. 6A to Fig. 6D). The second medical liquid spray is completed at a position where the piston 37 is in contact with the inner surface 200 located on the proximal side of the distal end part of the barrel 2.

According to the aforementioned syringe 1, the first pushing part 51 as a separate part from the lock part 50 of the rod side member 5 is pushed against the first pushed part 31 of the rod 3 in the first state. Thus, when the rod side member 5 is pushed by the user in order to carry out the pushing of the rod 3 to the barrel 2 in the first state, the first pushing part 51 is pushed against the first pushed part 31 of the rod 3 to move the rod 3 securely to the distal side in the axial direction. Thus, the first administration (in the syringe 1 of this embodiment, the first medical liquid spray) can be performed. Further, when the rod 3 is pushed from the first state to a predetermined position in the barrel 2, the lock between the lock part 50 of the rod side member 5 and the first lock part 30 of the rod 3 is released by the lock-release part 40 of the barrel side member 4. Accordingly, the rod side member 5 moves to the proximal side in the axial direction by the biasing force of the biasing member 6, thereby shifting into the second state. The second pushing part 52 as a separate part from the lock part 50 of the rod side member 5 is pushed against the second pushed part 32 of the rod 3 in the second state. Thus, when the rod side member 5 is pushed by the user in order to carry out the pushing of the rod 3 to the barrel 2 in the second state, the second pushing part 52 of the rod side member 5 is pushed against the second pushed part 32 of the rod 3 to move the rod 3 securely to the distal side in the axial direction. Thus, the second administration (in the syringe 1 of this embodiment, the second medical liquid spray) can be performed.

Since the first pushing part 51 and the second pushing part 52 as the separate parts from the lock part 50 of the rod side member 5 are pushed against the first pushed part 31 and the second pushed part 32 of the rod 3 as described above, the rod 3 can be pushed securely in the first and second administrations. Thus, two-part administration can be securely performed.

According to the syringe 1 of this embodiment having a structure requiring only the attaching of the barrel side member 4 and the rod side member 5 to the barrel 2 and the rod 3, the structure of the syringe 1 can be simplified. The barrel side member 4 can be applied also to the barrel 2 having a conventionally known shape with no need to form the barrel 2 into a special shape.

In the syringe 1 of this embodiment, in the second state, the pushing surface 521 of the second pushing part 52 and the second pushed surface 320 of the second pushed part 32 are pushed against each other with the flat surface to flat surface contact, and further, are held to be located at a position where the flat pushing surface 521 can come into contact with the flat second pushed surface 320 from the proximal side in the axial direction by the elasticity of the elastic part 520. Thereby, the second pushing part 52 can be securely pushed against the second pushed part 32.

Hereinafter, a container according to a second embodiment of the present invention will be described with reference to Fig. 7 and Fig. 8. The difference between this syringe 1 and the syringe 1 according to the first embodiment merely lies in the configuration of the lock piece 501 of the lock part 50 of the rod side member 5. The lock piece 501 has, in addition to the lock surface 502, the first outer surface 503, the second outer surface 504, and the third outer surface 505, a preventing surface 507 that provides connection between the lock surface 502 and the first outer surface 503 and is formed along the axial direction. This preventing surface 507 is a surface rising from the lock surface 502 to the proximal side. Accordingly, a portion including the lock surface 502 is a recess surrounded by the lock surface 502, the preventing surface 507, and the lock elastic part 500 in the lock piece 501 according to the second embodiment.

In the first state, the distal end part of the first lock part 30 of the rod 3 is fitted into the recess, and the lock surface 502 comes into contact with the first lock surface 300 of the first lock part 30 (see Fig. 7). The preventing surface 507 is located at a position where the preventing surface 507 can come into contact with the outer surface of the first lock part 30. Therefore, when the lock part 50 is moved radially inward unintentionally, the preventing surface 507 comes into contact with the outer surface of the first lock part 30, to thereby restrict the radially inward movement of the lock part 50 so as not to release the lock between the lock part 50 and the first lock part 30.

At the time of switching from the first state to the second state, the distal end part of the first lock part 30 comes out of the recess, to enhance the first outer surface 503 of the tapered surface to release the lock in the same manner as the syringe 1 of the first embodiment.

Since the second lock part 34 of the rod 3 is not fitted into the recess in the second state, the lock surface 502 does not come into contact with the first lock surface 340 of the second lock part 34. In this configuration, a pointed part 508 formed by the preventing surface 507 and the first lock surface 503 does not come into contact with the first lock surface 340 (see Fig. 8). In the second state, the second lock part 34 of the rod 3 is not fitted into the recess, and thus, it can be configured such that the lock surface 502 does not come into contact with the first lock surface 340 of the second lock part 34, and the pointed part 508 formed by the preventing surface 507 and the first lock surface 503 does not come into contact with the first lock surface 340. The syringe 1 including the lock piece 501 can be configured such that the second lock part 34 is fitted into the recess, and the lock surface 502 comes into contact with the first lock surface 340.

It is a matter of course that the syringe of the present invention is not limited to the aforementioned embodiment, but various modifications can be made without departing from the gist of the present invention. For example, a configuration of an embodiment can be added to a configuration of another embodiment, and part of a configuration of an embodiment can be replaced by a configuration of another embodiment. Further, part of a configuration of an embodiment can be deleted.

For example, the configuration of each member or part of the syringe 1 can be different from the aforementioned configuration.

Specifically, the rod side member 5 may not include at least one of the third pushing part 53 and the extending part 56 as long as the rod side member 5 includes the lock part 50, the first pushing part 51, and the second pushing part 52.

The second pushing part 52 includes the elastic part 520 and the second pushing surface part 522, but can have a different configuration. For example, the second pushing part 52 can be formed only by the elastically deformable second pushing surface part 522.

The rod 3 may not include at least one of the third pushed part 33 and the second lock part 34 as long as the rod 3 includes the first lock part 30, the first pushed part 31, and the second pushed part 32.

In the syringe 1 of the aforementioned embodiment, the first pushed part 31 is a proximal end part of the rod 3, but not limited to this configuration. For example, the first pushed part 31 can be provided with a recess extending from the proximal side of the rod 3 to the distal side in the axial direction of the rod 3, in which the bottom of this recess serves as the first pushed surface 310, to allow the first pushing part 51 of the rod side member 5 to be inserted into the recess and pushed against the first pushed surface 310.

In the syringe 1 of the aforementioned embodiment, the barrel side member 4 is fitted onto the flange part 23 of the barrel 2, but can be attached to the barrel 2 by another way of attachment other than the fitting engagement. For example, the barrel side member 4 can be attached to the barrel body 22.

Further, the barrel side member 4 may not include, for example, the finger hook part 42 as long as the barrel side member 4 includes the lock-release part 40. The barrel 2 may not include the flange part 23.

The syringe 1 may not include the push restriction mechanism 8. The syringe 1 may not include the injection nozzle 7. In this case, it is conceivable that the syringe 1 discharge the content (e.g., medical liquid) not in the form of mist but in a linear manner from the distal end part 20 of the barrel 2. Note that, in this case, the rod 3 can have no pressure accumulating projection 351.

Each functional part in the syringe 1 can have another shape with an equivalent function.

As described above, according to the present invention, a syringe capable of dividing a content to be administered in two parts and securing the two-part administration can be provided.

The syringe of the present invention is a syringe for dividing a content to be administered in plural parts, the syringe including: a barrel having a distal end part and a proximal end part in an axial direction and allowed to discharge the content from the distal end part; a rod configured to be inserted into the barrel from a proximal side in the axial direction and pushed to a distal side; a barrel side member attached to the barrel; a rod side member attached to the rod and configured to be shifted between a first state of being locked at a predetermined position of the rod in the axial direction and a second state of being located at a position on the proximal side in the axial direction of the first state; and a biasing member configured to be locked on the rod and the rod side member to bias the rod side member to move it from the first state toward the second state, the rod side member including: a lock part configured to be locked to the rod in the axial direction in the first state; a first pushing part configured to be pushed against the rod toward the distal side in the axial direction in the first state, and be a part separate from the lock part; and a second pushing part configured to be pushed against the rod toward the distal side in the axial direction in the second state, and be a part separate from the lock part; the rod including: a first lock part to which the lock part is locked in the first state; a first pushed part against which the first pushing part is pushed in the first state; and a second pushed part against which the second pushing part is pushed in the second state, and the barrel side member including: a lock-release part configured to release lock between the lock part and the first lock part with the rod pushed to the predetermined position in the barrel in the first state.

According to such a configuration, in the first state where the first pushing part as a part different from the lock part is pushed against the first pushed part, when the rod side member is pushed in order to carry out the pushing operation of the rod to the barrel in the first state, the first pushing part is pushed against the first pushed part to move the rod securely to the distal side in the axial direction. Thus, the first administration can be performed. Further, when the rod is pushed from the first state to the predetermined position, the lock of the lock part and the first lock part is released by the lock-release part. Consequently, the rod side member moves the proximal side in the axial direction by the biasing force of the biasing member, thereby going into the second state. In the second state where the second pushing part as a part different from the lock part is pushed against the second pushed part, when the rod side member is pushed in order to carry out the pushing operation of the rod to the barrel in the second state, the second pushing part is pushed against the second pushed part to move the rod securely to the distal side in the axial direction. Thus, the second administration can be performed. Since the first pushing part and the second pushing part each being a part different from the lock part of the rod side member are pushed against the first pushed part and the second pushed part of the rod as described above, the rod can be securely pushed in the first and second administrations, thereby securing the twice administration.

In the syringe, it can be configured such that the barrel includes a barrel body and a flange part disposed in the barrel body, the barrel side member is fitted onto the flange part to be attached to the barrel, the rod includes a rod body and a member attachment part disposed in the rod body, and the rod side member is attached to the member attachment part.

According to the syringe having the structure requiring only the attaching of the barrel side member and the rod side member to the barrel and the rod, the structure of the syringe can be simplified. The barrel side member can be applied also to the barrel having a conventionally known shape with no need to form the barrel into a special shape.

In the syringe, it can be configured such that the second pushing part includes: an elastic part having elasticity in a radial direction perpendicular to the axial direction; and a pushing surface part disposed in the elastic part, and having a pushing surface facing the distal side in the axial direction and being a flat surface perpendicular to the axial direction, the second pushed part includes: a pushed surface part having a pushed surface facing the proximal side in the axial direction and being a flat surface perpendicular to the axial direction, and the pushing surface is held, in the second state, by the elasticity of the elastic part at a position capable of coming into contact with the pushed surface from the proximal side in the axial direction.

According to such a configuration, in the second state, the surfaces are pushed against each other with the flat surface to flat surface contact, and further, are held to be located at a position where the pushing surface can come into abutting contact with the pushed surface from the proximal side in the axial direction by the elasticity of the elastic part. Thereby, the second pushing part can be securely pushed against the second pushed part.

### REFERENCE SIGNS LIST

1: Syringe
2: Barrel
3. Rod
4: Barrel side member
5: Rod side member
6: Biasing member
7: Injection nozzle
8: Push restriction mechanism
20: Distal end part
21: Proximal end part
22: Barrel body
23: Flange part
30: First lock part
31: First pushed part
32: Second pushed part
33: Third pushed part
34: Second lock part
35: Rod body
36: Member attachment part
37: Piston
40: Lock-release part
41: Barrel attachment part
42: Finger hook part
43: Barrel side body
44: Restriction part
50: Lock part
51: First pushing part
52: Second pushing part
53: Third pushing part
54: Top panel part
55: Skirt part
56: Extending part
57: Contact part
100: Nasal cavity administration container
101: Barrel
102: Plunger
103: Plunger rod
105: Elastic member
200: Inner surface
220: Body distal end part
221: Body proximal end part
222: Body connection part
300: First lock surface
301: First through-hole
310: First pushed surface
320: Second pushed surface (pushed surface)
321: Second pushed surface part (pushed surface part)
330: Third pushed surface
340: Second lock surface
341: Second through-hole
350: Shaft part
351: Pressure accumulating projection
351a: Proximal pressure accumulating projection
351b: Distal pressure accumulating projection
360: Widened part
361: Insertion hole
370: Distal end surface
340: Second lock surface
400: Inclined surface
410: Attachment part through-hole
411: Pressure accumulating part
440: Restriction surface
500: Lock elastic part
501: Lock piece
502: Lock surface
503: First outer surface
504: Second outer surface
505: Third outer surface
506: Outer surface
507: Preventing surface
508: Pointed part
510: Extending part
511: First pushing surface
512: First pushing surface part
520: Elastic part
521: Second pushing surface (pushing surface)
522: Second pushing surface part (pushing surface part)
523: Inner circumferential surface
524: Proximal side inner circumferential surface
525: Connecting inner circumferential surface
530: Third pushing surface
540: Outer circumferential portion
570: Contact surface
1016: Projecting part
1016a: Projecting part
1020b: Other end
1026: First elongated hole
1028: Second elongated hole
1030b: Other end
1030L: Leaf spring
1034: Pushing part
1034a: Surface
1036: First lock part
1036a: Front end part
1038: Second lock part
AR2, AR4: Arrow

## Claims

1. A syringe for dividing a content to be administered in plural parts, the syringe comprising:
a barrel having a distal end part and a proximal end part in an axial direction and allowed to discharge the content from the distal end part;
a rod configured to be inserted into the barrel from a proximal side in the axial direction and pushed to a distal side;
a barrel side member attached to the barrel;
a rod side member attached to the rod and configured to be shifted between a first state of being locked at a predetermined position of the rod in the axial direction and a second state of being located at a position on the proximal side in the axial direction of the first state; and
a biasing member configured to be locked on the rod and the rod side member to bias the rod side member to move it from the first state toward the second state,
the rod side member comprising:
a lock part configured to be locked to the rod in the axial direction in the first state;
a first pushing part configured to be pushed against the rod toward the distal side in the axial direction in the first state, and be a part separate from the lock part; and
a second pushing part configured to be pushed against the rod toward the distal side in the axial direction in the second state, and be a part separate from the lock part;
the rod comprising:
a first lock part to which the lock part is locked in the first state;
a first pushed part against which the first pushing part is pushed in the first state; and
a second pushed part against which the second pushing part is pushed in the second state, and
the barrel side member comprising:
a lock-release part configured to release lock between the lock part and the first lock part with the rod pushed to the predetermined position in the barrel in the first state.

2. The syringe according to claim 1, wherein
the barrel comprises a barrel body and a flange part disposed in the barrel body,
the barrel side member is fitted onto the flange part to be attached to the barrel,
the rod comprises a rod body and a member attachment part disposed in the rod body, and
the rod side member is attached to the member attachment part.

3. The syringe according to claim 1 or 2, wherein
the second pushing part comprises:
an elastic part having elasticity in a radial direction perpendicular to the axial direction; and
a pushing surface part disposed in the elastic part, and having a pushing surface facing the distal side in the axial direction and being a flat surface perpendicular to the axial direction,
the second pushed part comprises:
a pushed surface part having a pushed surface facing the proximal side in the axial direction and being a flat surface perpendicular to the axial direction, and
the pushing surface is held, in the second state, by the elasticity of the elastic part at a position capable of coming into contact with the pushed surface from the proximal side in the axial direction.
